Europäisches Patentamt

European Patent Office

Office européen des brevets

(11) Veröffentlichungsnummer: **0 024 048**
**B1**

(12) **EUROPÄISCHE PATENTSCHRIFT**

(45) Veröffentlichungstag der Patentschrift:
16.05.84

(21) Anmeldenummer: 80104700.2

(22) Anmeldetag: 09.08.80

(51) Int. Cl.³: **C 12 M 1/12,** C 12 M 1/20

(54) Zweiseitig beschichtbares Membranfilter zum Einsatz im mikrobiologischen Screening.

(30) Priorität: 11.08.79 DE 2932694

(43) Veröffentlichungstag der Anmeldung:
18.02.81 Patentblatt 81/7

(45) Bekanntmachung des Hinweises auf die Patenterteilung:
16.05.84 Patentblatt 84/20

(84) Benannte Vertragsstaaten:
CH FR GB LI

(56) Entgegenhaltungen:
DE - C - 839 245
US - A - 2 672 432
US - A - 3 221 878

(73) Patentinhaber: Breuker, Eberhard, Dr.,
Rektenstrasse 10, D-4930 Detmold (DE)

(72) Erfinder: Breuker, Eberhard, Dr., Rektenstrasse 10,
D-4930 Detmold (DE)

(74) Vertreter: Redies, Bernd, Dr. rer. nat. et al, Redies,
Redies, Türk & Gille Brucknerstrasse 20,
D-4000 Düsseldorf 13 (DE)

BUNDESDRUCKEREI BERLIN

## Zweiseitig beschichtbares Membranfilter zum Einsatz im mikrobiologischen Screening

Die Erfindung betrifft die durch die Patentansprüche definierten Gegenstände.

Im mikrobiologischen Screening ist es oft notwendig, Mikroorganismen zu erkennen, die bestimmte Wirkstoffe produzieren (Antibiotika, Enzyme, Vitamine). Oft ist das Modell zum Wirkungsnachweis auch ein mikrobiologisches System, zum Beispiel der Nachweis der Antibiotika durch Testorganismen.

Für diesen konkreten Fall sind in der Vergangenheit verschiedene Konzeptionen realisiert worden. Eine Möglichkeit ist die Überschichtung einer Platte, die wirkstoffproduzierende Organismen in Kolonieform hat, mit einer zweiten Agarschicht, die den Testkeim enthält. Die Antibiotikawirkung kann dann an einem Hemmhof im Umfeld der Pilzkolonie erkannt werden. Diese Methode besitzt jedoch bei der Anwendung in der Praxis die folgenden Nachteile;

1. Schmiereffekt beim Überschichten der zu testenden Organismen.
2. Schlechte Isoliermöglichkeiten nach Überschichtung.

Vor allem der Schmiereffekt stellt die Brauchbarkeit dieser Methode sehr infrage. Beim Überschichten läßt sich kaum vermeiden, daß die Sporen bzw. Konidien (Zellen) von den zu prüfenden Kolonien auf der Platte verteilt werden und wieder anwachsen. Eine eindeutige Zuordnung von Hemmöfen zu den verschiedenen Pilzen, bzw. Bakterienkolonien ist dann nicht mehr möglich.

Bei einem mikrobiologischen Screening, speziell Antibiotika-Screening, stellt sich vor allem das Problem aus einer Vielzahl von isolierten Organismen, die zu erkennen, die bestimmte Wirkstoffe, zum Beispiel Antibiotika, produzieren. Einen Nährboden nur auf einer Seite anzuordnen, wie in der US-PS 26 72 432 beschrieben, ist hierfür ungeeignet, da es zu lange dauern würde, bis — und sogar unsicher wäre, daß — genügend Wirkstoff in das horizontal seitlich gelegene Feld und dem dort wachsenden Mikroorganismus wandert. Flüssige Nährmedien auf zwei Seiten einer Membran zu verwenden, wie in der US-PS 32 21 878 beschrieben, ist für das mikrobiologische Screening nicht möglich, da sich so die zu testenden Mikroorganismen nicht genügend eindeutig lokalisieren und so identifizieren lassen.

Die Fig. I und II zeigen die erfindungsgemäße Vorrichtung in zwei Beispielen im Schnitt.

Die Vorrichtung besteht aus einer Haltevorrichtung, beispielsweise dem Hohlkörper (1), der z. B. ein Rohrstück sein kann, welches in einem genügenden Abstand zu der Bodenplatte (6), eine Halterung für ein Gitter, ein Netz oder eine Lochplatte (3) besitzt. Gemäß der Ausführung in Fig. II liegt das Netz bzw. die Lochplatte (3) auf der unteren Hohlkörperform (Rohrstück) (1b) auf, während die zweite Hohlkörperform (1a) auf dem Filter und/oder dem Netz bzw. der Lochplatte (3) aufsitzt. Auf der Lochplatte (3) liegt das bakteriendichte Membranfilter (2) und darauf ist die Agar-Schicht (4) aufgetragen. Unter der Lochplatte (3) ist die zweite Agar-Schicht (5) aufgetragen.

Das Netz bzw. die Lochplatte (3) ist nicht unbedingt notwendig, sondern das Membranfilter kann auch ohne eine solche Hilfsvorrichtung in der Haltevorrichtung befestigt bzw. eingespannt werden.

Die Hohlform (1) bzw. die Hohlformstücke (1a bzw. 1b) können aus Metall (beispielsweise Aluminium) oder auch aus einem anderen Material (zum Beispiel Polystyrol, Polypropylen, Edelstahl, Teflon®) sein. Der Durchmesser liegt zweckmäßig zwischen 2—20 cm.

Beispielsweise kann es sich bei den Hohlformstücken (1a) und (1b) um Metallringe (Aluminiumringe) oder Kunststoffringe handeln.

Die Halterung für das Netz, Gitter oder die Lochplatte (3) bzw. für das Filter (2) (falls die Vorrichtung gemäß (3) nicht vorgesehen ist) in der Hohlform (1) ist zweckmäßig in einem Abstand von 1 bis 3 cm von der Bodenplatte (6) angebracht; falls die Haltevorrichtung zum Beispiel aus zwei Rohrstücken besteht, beträgt die Höhe des unteren Rohrstückes (1b) beispielsweise zwischen 1 und 3 cm, vorzugsweise 2 cm. Das daraufliegende Rohrstück (1a) besitzt die gleiche Höhe bzw. kann gegebenenfalls auch in der Höhe von dem unteren Rohrstück (1b) abweichen. Es muß jedoch auf jeden Fall zwischen dem oberen Rand der Agar-Schicht und dem oberen Ende der Hohlform (1a) ein Mindestabstand von 0,5 cm liegen. Die Wandstärke der Hohlform beträgt beispielsweise zwischen 0,2 und 1,0 cm; falls die Haltevorrichtung aus zwei Hohlformen (Rohrstücken) entsprechend Fig. II besteht, ist die Wandstärke beider Hohlformen zweckmäßig etwas dicker, beispielsweise zwischen 0,5—2,0 cm.

Die untere Bodenplatte (6) ist vorzugsweise eine Glasplatte. Die erfindungsgemäße Vorrichtung kann oben ebenfalls mit einer Glasplatte abgedeckt werden. Es ist jedoch auch möglich, die ganze Vorrichtung in einen Glasbehälter, der oben mit einer Glasplatte abschließbar ist, zu stellen, bzw. statt Glasplatten durchsichtige Kunststoffplatten z. B. aus Polystyrol oder Kunststoffdeckel zu verwenden.

Die Lochplatte bzw. das Netz oder Gitter (3) besteht aus Metall (zum Beispiel Aluminium oder Edelstahl) oder einem Kunststoff (wie zum Beispiel Polystyrol z. B. beim Einsatz von Einweg-Screningplatten ähnlich dem Einsatz von Einweg-Petrischalen in der Bakteriologie). Die Dikke der Lochplatte, des Netzes oder Gitters kann zwischen 0,1—0,5 cm liegen.

Falls es sich bei der Vorrichtung (3) um eine Lochplatte handelt, dann sind die Abstände zwischen den einzelnen Löchern möglichst klein (z. B. 0,1—0,2 cm) und der Durchmesser der Löcher möglichst groß (z. B. zwischen 1—1,5 cm). Im allgemeinen enthält die Lochplatte auf 1 cm² beispielsweise 1 Loch mit einem Durchmesser von 1 cm.

Die Form der Löcher spielt keine Rolle. Vorzugsweise sind diese rund.

Als Membranfilter (2) ist jedes bakteriendichte Membranfilter, welches sterilisierbar ist, geeignet. Zum Beispiel sind in der DE-PS 8 39 245 Membranfilter beschrieben, die auch sterilisierbar sind. In der dort beschriebenen Form und Anordnung sind sie für die Durchführung des mikrobiologischen Screenings nicht geeignet. Unter Bakteriendichte versteht man Porengrößen von 0,22 bis 0,45 μ. Die Art und Gestaltung des Filters ist ohne große Bedeutung. Es muß nur gewährleistet sein, daß zwischen den beiden Agar-Schichten (4) und (5) (Agar-Medium),

1. für den Wirkstoffproduzenten auf der einen Seite,
2. für den Teststamm auf der anderen Seite,

ein unmittelbarer wäßriger Kontakt durch das Filter gegeben ist. Durch diesen Kontakt ist gewährleistet, daß die nachzuweisenden Wirkstoffe in das Testsystem diffundieren können. Allgemein kann festgehalten werden, daß Filter, die für wäßrige Medien geeignet sind und ohne baktericide Rückstände sterilisierbar sind, in Frage kommen.

Die eine der beiden Agar-Schichten (4) oder (5) dient zur Aufnahme der wirkstoffproduzierenden Organismen, die auf dieser Agar-Schicht in Kolonien wachsen können, beispielsweise aus einer Verdünnungsreihe. Im allgemeinen wird man die obere Agar-Schicht (4) für die wirkstoffproduzierenden Organismen verwenden. Hierbei kann jeder Nährboden, der für den Wirkstoffproduzenten und für die Wirkstoffproduktion optimal ist, als Agar-Schicht aufgetragen werden. Ebenso jeder Nährboden der für den Testkeim optimal ist. Diese Möglichkeit ist ein großer Vorzug der erfindungsgemäßen Vorrichtung. Wirkstoffproduzent und Testkeim können im eigenen optimalen Nährboden zum Wachstum bzw. zur Wirkstoffproduktion gebracht werden. Die Agar-Nährböden selbst sollen die übliche Agar-Konzentration von 1% bis 2%, vorzugsweise 1,5% besitzen. Hierdurch ist einerseits eine ausreichende Konsistenz für die notwendige Stabilität der Agar-Schicht und andererseits genügend freies Wasser für das Wachstum der Mikroorganismen vorhanden. Die Schichtdicke der beiden Agar-Schichten (4) und (5) ist nicht kritisch. Sie liegt in der Größenordnung, wie sie normalerweise zum Beispiel in Petrischalen zur Kultivierung von Mikroorganismen üblich ist. Sie beträgt beispielsweise 0,2 bis 0,4 cm, vorzugsweise 0,3 bis 0,4 cm.

Bei den Agar-Schichten handelt es sich um die in der Mikrobiologie üblicherweise verwendeten Nährböden, wie sie beispielsweise in den Firmen

| Merck, Darmstadt | z. B. Nähragar |
| Oxoid, Wesel | z. B. Casoagar |
| Difco, Hamburg | z. B. Würzeagar |

angegeben sind. Die Agar-Schichten (4) und (5) können gleich, sie können aber auch verschieden sein. Als Agar-Schichten für die Wirkstoffproduzenten kommen insbesondere folgende Nährböden in Betracht: Spezialnährböden für verschiedene Pilzarten, z. B. Streptomyceten. Literatur: Anreicherungskultur und Mutantenauslese Symposium 1964, Göttingen, Gust. Fischer, Stuttgart, Seiten 228—252. Als Agar-Böden für die Testkeime können beispielsweise folgende Nährböden in Betracht: Nähragar, Casoagar.

Die nachzuweisenden wirkstoffproduzierenden Organismen werden z. B. aus einer Screening-Bodenprobe über eine Verdünnungsreihe auf die Agarschicht (4) gebracht. Auf der Agarschicht (4) sollen zweckmäßigerweise 5—10 Kolonien, von Wirkstoffproduzenten vorhanden sein.

Die Testkeime werden im allgemeinen in die untere Agar-Schicht (5) gegeben, zweckmäßig vor dem Auftragen dieser Agar-Schicht.

Die von den Mikroorganismen produzierten Wirkstoffe diffundieren von der einen Agar-Schicht (beispielsweise der Agar-Schicht (4), in die zweite Agar-Schicht (beispielsweise Agar-Schicht (5)) und kommen dort zur Wirkung, das heißt, es bilden sich auf der Agar-Schicht die den bzw. die Testkeime enthält ein bzw. mehrere Hemmhöfe aus. Dabei ist es zweckmäßig, das Membranfilter in der Porengröße so zu wählen (zum Beispiel 0,2 μm), daß ein Wachstum der Organismen von der einen in die andere Schicht verhindert wird.

Dadurch wird ein Überstrich oder Ineinanderwachsen von wirkstoffproduzierendem Stamm und Testkeim verhindert.

Die erfindungsgemäße Vorrichtung ist beispielsweise rund. Selbstverständlich ist auch ein quadratischer oder ein anderer Querschnitt möglich. Von unten wird die Größe des Membranfilters bzw. des Netzes, Gitters bzw. der Lochplatte von der Koloniegröße der wirkstoffproduzierenden Kultur begrenzt, daß heißt, im allgemeinen liegt die untere Grenze bei einem Durchmesser von 1 bis 2 cm. In diesem Bereich ist makroskopisch auf der anderen Membranseite im Tastagar noch ein Einfluß eines Wirkstoffes auf die Testkeime erkennbar. Nach oben ist die Filtergröße bzw. Lochplatten-, Gitter- oder Netzgröße konstruktiv durch die Stabilität dieser Materialien und sowie durch deren Handlichkeit begrenzt. Ein vernünftiger Durchmesser liegt zum Beispiel bei 20 cm. Auf einem solchen Filter können ca. 7—9 wirkstoffproduzierende Kolonien gleichzeitig getestet werden.

Die Isolierung der als Wirkstoffproduzent erkannten Kolonie ist in der hierfür üblichen Art leicht und ohne Schwierigkeit möglich.

Im allgemeinen wird das mikrobiologische Screening wie folgt durchgeführt: Die eine Agar-Schicht der erfindungsgemäßen Vorrichtung

wird mit den verschiedenen Kulturen von Mikroorganismen geimpft und dann die Vorrichtung nach Abdeckung mit zwei sterilen Glasplatten oder Einsetzen in einen verschließbaren sterilen Behälter in üblicher Weise mehrere Tage (7—10 Tage) bei Temperaturen zwischen 15—35°C (zum Beispiel im Brutschrank) bebrütet. Dann wird die 2. Agar-Schicht, die den Testkeim enthält, auf der anderen Membranseite aufgebracht und wieder bebrütet, dieses Mal bei Temperaturen zwischen 15—40°C. Diese zweite Bebrütungsphase dauert im allgemeinen nur 24 Stunden bis 2 Tage.

Anschließend erkennt man an der den Testkeim enthaltenden Agar-Schicht anhand der Ausbildung von sogenannten Hemmhöfen ob und welche Kolonien der mit den Mikroorganismen geimpften Agar-Schicht Wirkstoffe produziert haben.

Das mikrobiologische Screening ist insbesondere geeignet für Organismen, die antibiotische Wirkstoffe produzieren. Das erfindungsgemäße Testsystem kann beim Screening neuer Antibiotikabildner, zum Beispiel in der Weise eingesetzt werden, daß als Teststamm solche verwandt wurden, die gegen bekannte Antibiotika resistent sind. So werden von vornherein solche Antibiotikabildner erkannt, die einen Wirkstoff produzieren, der mit den bekannten nicht identisch ist.

Das erfindungsgemäße System kann weiterhin zum Beispiel eingesetzt werden bei der Suche nach mikrobiellen Aminosäureproduzenten und Vitaminproduzenten, bzw. beim Screening vor Hochleistungsmutanten für die Fermentation. Weitere Anwendungen liegen zum Beispiel im Forschungsbereich, zum Beispiel in der Pilz- oder Bakteriengenetik, wenn es darum geht, Mutanten, die bestimmte Stoffwechselprodukte ausscheiden bzw. anreichern, oder zum Beispiel Defekt-Mutanten zu erkennen oder im analytischen Bereich, wenn es zum Beispiel darum geht, die mutagene Wirkung von Mykotoxinen, die von Lebensmittelkontaminaten produziert werden, mit Hilfe eines bakteriellen Testes, zum Beispiel Ames-Test, zu erkennen.

Weiterhin bedeutet die erfindungsgemäße Vorrichtung eine wesentliche Verbesserung bei der Erkennung von pathogenen Stphylokokken in der Lebensmittelindustrie (Fleischverarbeitung) und in der Medizin (Hospitalismus).

Die erfindungsgemäße Vorrichtung bzw. das mit Hilfe hiervon durchgeführte mikrobiologische Screening-Verfahren besitzt gegenüber den bisher bekannten Methoden die folgenden Vorzüge:

1. Wirkstoffproduzent und Testorganismus sind mikrobiologisch voneinander getrennt, d. h.: sie können jeder in einem speziellen Nährmedium kultiviert werden:
Der Wirkstoffproduzent in einem Medium, das für die Wirkstoff- oder Metabolitenproduktion optimal ist, der Teststamm in einem Medium, das für den Test optimal ist.

2. Der Nachweis der Wirkstoffe ist direkt durch Diffusion in den Testagar möglich, das heißt: somit der Nachweis des Wirkstoffproduzenten.

3. Eine mikrobiologische saubere Abimpfung der Wirkstoffproduzenten (Pilze oder Bakterien) ist jederzeit möglich.

4. Beim Anlegen der 2. Schicht kommt es nicht zu Schmiereffekten mit der 1. Schicht.

Beispiel

Einsatz der erfindungsgemäßen Vorrichtung im Antibiotikascreening

Vier verschiedene Pilzstämme, — Isolierung als Waldböden — wurden mit der erfindungsgemäßen Vorrichtung auf ihre Fähigkeit untersucht, Metaboliten mit antiobiotischer Wirkung zu produzieren.

Die obere Seite einer Vorrichtung gemäß Fig.-II mit eingespanntem und sterilisiertem Membranfilter (Fa. Sartorius; 25 g, Type SM 11 607, Format 150, Porengröße 0,2 μm wurde mit dem Spezial Screeningnährboden SM1 beschichtet. Die Nährbodenmenge betrug 75 ml. Der Spezialnährboden enthielt als wesentliche Bestandteile Cornsteep Liquor, Sojamehl, Glyzerin und $CaCO_3$. Der Agar-Anteil betrug 1,5%. Nach dem Erkalten wurde der Agar mit den Stämmen 71, 136, 135 und 68 von einer 3—4 Tage alten Schrägröhrchenkultur, die einen ähnlichen Agar enthielt, beimpft. Die Vorrichtung wurde anschließend mit zwei sterilen Glasplatten je 20 · 20 cm, auf beiden Seiten abgedeckt und im Brutschrank bei 25°C, einer günstigen Temperatur für das Wachstum und die Antiobiotikabildung, 10 Tage bebrütet. Da die meisten Antibiotika gegen Ende der Logarithmischen Wachstumsphase bzw. in der stationären Phase gebildet werden, war zu erwarten, daß zu diesem Zeitpunkt möglicherweise Antibiotika vorhanden waren. Der Zeitpunkt zum Nachweis der gebildeten Antibiotika erschien somit angezeigt.

Als Teststamm zum Nachweis produzierter Antibiotika wurde ein gramnegativer Keim aus der Gattung Serratia marcescens eingesetzt. Die Gramnegativen Keime sind im Zusammenhang mit dem Hospitalismus von großer Bedeutung. Neue Antibiotika gegen diese Erreger sind daher von großem Nutzen.

75 ml Nähragar (Merck Art. Nr. 5450, Fleischextrakt 3,0 g p.l. Pepton aus Fleisch 5,0 g p.l. und Agar-Agar 12,0 g p.l.) wurden nach dem Lösen auf 45°C gebracht, mit 0,2 ml einer Serratia Impfsuspension beimpft, durchmischt und auf die andere Seite der Vorrichtung gegossen. Nach dem Erkalten wurde die beidseitig abgedeckte »Doppelschale« 24 Stunden bei 35°C bebrütet. Die Impfsuspension wird durch 24stündiges Schütteln von Serratia marcescens mit 5 ml Nährbouillon, erhalten.

Es zeigte sich in der Testagarschicht ein klarer Hemmhof von ca. 3,5 cm Durchmesser, der genau gegenüber der Kolonie des Pilzstammes Nr. 71 lag. Dies zeigt an, daß dieser Pilzstamm Nr. 71 eine Verbindung produziert und ausgeschieden hat, die nach Diffusion in den Testagar während der Bebrütung bei 35°C das Wachstum des Testkeimes an dieser Stelle verhindert hat.

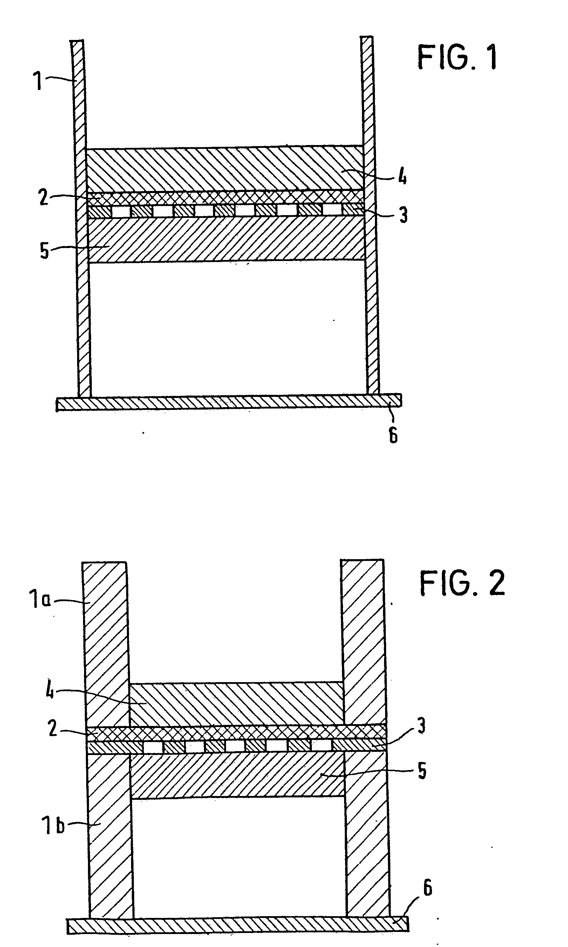

## Patentansprüche

1. Vorrichtung zum Erkennen von Mikroorganismen, die bestimmte Wirkstoffe produzieren, für den Einsatz beim mikrobiologischen Screening, mit einem Membranfilter und mindestens einer damit in Kontakt stehenden Agar-Schicht, welche zur Aufnahme der wirkstoffproduzierenden Mikroorganismen sowie Testkeime dient, dadurch gekennzeichnet, daß das in einer Haltevorrichtung (1, 3) befestigte Membranfilter (2) auf seiner Ober- und Unterseite jeweils eine Agar-Schicht (4, 5) aufweist, von denen die eine zur Aufnahme der wirkstoffproduzierenden Mikroorganismen und die andere zur Aufnahme der Testkeime bestimmt ist.

2. Vorrichtung nach Anspruch 1, dadurch gekennzeichnet, daß der Teil (1) der Haltevorrichtung (1, 3), in dem das Membranfilter (2) bzw. der als Gitter, Netz oder Lochplatte ausgebildete zweite Teil (3) der Haltevorrichtung befestigt ist, einen Hohlkörper (1a) darstellt.

3. Vorrichtung nach einem oder mehreren der vorangegangenen Ansprüche, dadurch gekennzeichnet, daß der Teil (1) der Haltevorrichtung aus zwei Hohlkörperstücken (1a, 1b) besteht, wobei das Membranfilter (2) und/oder das Netz (3), das Gitter oder die Lochplatte auf dem einen Hohlkörperstück (1b) aufliegt und das zweite Hohlkörperstück (1a) auf dem Membranfilter aufliegt.

## Claims

1. Apparatus for detecting microorganisms producing certain effective substances for use in microbiological screening, comprising a diaphragm filter and at least one contacting agar layer receiving the effective substance producing microorganisms, as well as test germs, characterized in that diaphragm filter (2) fixed in a support (1, 3) comprises on each of its upper and lower surfaces an agar layer (4, 5) one of which receives the effective substance producing microorganisms while the other of said surfaces receives the test germs.

2. Apparatus as defined in claim 1, characterized in that portion (1) of support device (1, 3) in which is fixed diaphragm filter (2) or the second portion (3) auf the supoort device designed as a grid, net or perforated plate, represents a hollow body (1a).

3. Apparatus as defined in one or several of the preceding claims, characterized in that portion (1) of the support device comprises a pair of hollow parts (1a, 1b), said diaphragm filter (2) and/or plate sits on one of hollow parts (1b) while the other hollow part (1a) sits on the diaphragm filter.

## Revendications

1. Dispositif pour reconnaître les microorganismes qui produisent certaines substances actives aux fins d'utilisation dans le dépistage microbiologique, avec un filtre à membrane et au moins une couche de gélose restant en contact aved lui, servant à absorber les microorganismes producteurs de substances actives, ainsi que des germes expérimentaux, caractérisé en ce que le filtre à membrane (2) fixé dans un dispositif de retenue (1, 3) présente sur son côté supérieur comme sur son côté inférieur une couche de gélose (4, 5), dont l'une est prévue pour absorber les microorganismes producteurs de substances actives et l'autre pour absorber les germes expérimentaux.

2. Dispositif selon la revendication 1, caractérisé en ce que la partie (1) du dispositif de retenue (1, 3) dans lequel est fixé le filtre à membrane (2) ou la seconde partie (3) ayant la forme d'un grillage, d'un filet ou d'une plaque trouée du dispositif de retenue représente un corps creux (1a).

3. Dispositif selon ou plusieurs des revendications précédentes, caractérisé en ce que la partie (1) du dispositif de retenue se compose de deux pièces en corps creux (1a, 1b), le filtre à membrane (2) et/ou le filet (3), le grillage ou la plaque trouée se situant sur l'une des pièces en corps creux (1b) et la seconde pièce en corps creux (1a) se situant sur le filtre à membrane.

FIG. 1

FIG. 2